(19) **Europäisches Patentamt European Patent Office Office européen des brevets**

(11) **EP 4 059 410 B1**

(12) **EUROPEAN PATENT SPECIFICATION**

(45) Date of publication and mention
of the grant of the patent:
**26.11.2025 Bulletin 2025/48**

(51) International Patent Classification (IPC):
*A61B 5/00* (2006.01)     *A61B 5/0531* (2021.01)
*A61B 5/0537* (2021.01)     *H04R 5/033* (2006.01)

(21) Application number: **21163148.6**

(22) Date of filing: **17.03.2021**

(52) Cooperative Patent Classification (CPC):
**A61B 5/0531; A61B 5/0537; A61B 5/6817;**
**H04R 5/033**

(54) **ARRANGEMENT AND METHOD FOR MEASURING AN ELECTRICAL PROPERTY OF A BODY**

ANORDNUNG UND VERFAHREN ZUR MESSUNG EINER ELEKTRISCHEN EIGENSCHAFT EINES KÖRPERS

AGENCEMENT ET PROCÉDÉ POUR MESURER UNE PROPRIÉTÉ ÉLECTRIQUE D'UN CORPS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**21.09.2022 Bulletin 2022/38**

(73) Proprietor: **Sonova AG**
**8712 Stäfa (CH)**

(72) Inventors:
• **LEUTHOLD, Nathalie**
**8712 Stäfa (CH)**

• **PEREZ, Gabriel**
**8708 Männedorf (CH)**
• **RIEPENHOFF, Matthias**
**8634 Hombrechtikon (CH)**
• **BAER, Daniel**
**8004 Zürich (CH)**

(74) Representative: **Liedtke & Partner Patentanwälte**
**Gerhart-Hauptmann-Straße 10/11**
**99096 Erfurt (DE)**

(56) References cited:
EP-A1- 3 154 278     US-A1- 2005 250 996
US-A1- 2017 339 484     US-A1- 2019 053 736
US-A1- 2020 029 881     US-A1- 2020 268 260

## Description

Technical Field

The invention relates to an arrangement and a method for measuring an electrical property of a human or animal body.

Background of the Invention

US 2017/0078785 A1 discloses earbuds configured with one or more biometric sensors. At least one of the biometric sensors is configured to be pressed up against a portion of the tragus for making biometric measurements. In some embodiments, the housing of the earbud can be symmetric so that the earbud can be worn interchangeably in either a left or a right ear of a user. In such an embodiment, the earbud can include a sensor and circuitry configured to determine and alter operation of the earbud in accordance to which ear the earbud is determined to be positioned within.

EP 3 154 278 A1 describes a hearing system for a user, with a hearing device, in particular a BTE device, which has a microphone for detecting an audio signal, and with a control unit which is designed classify a physiological state of the user based on the audio signal. The audio signal is examined in particular with regard to characteristic features that enable specific physiologically relevant noises made by the user to be identified. The presence of symptoms which characterize a certain state is then derived from this.

Summary of the Invention

It is an object of the present invention to provide an improved arrangement and an improved method for measuring an electrical property of a human or animal body.

The object is achieved by an arrangement for measuring an electrical property of a human or animal body according to claim 1 and by a method for measuring an electrical property of a human or animal body according to claim 10.

Preferred embodiments of the invention are given in the dependent claims.

According to the invention an arrangement for measuring an electrical property of a human or animal body between a first position and a second position of the body is proposed, the arrangement comprising a first contact configured to contact the body at the first position and a second contact configured to contact the body at the second position, each contact comprising at least a first and a second electrode for contacting the body, the arrangement further comprising at least two measurement units configured to measure a resistance and/or impedance between the contacts and to measure a contact resistance between the two electrodes of each contact, respectively.

The measurement units may comprise a first and a second terminal. The second terminal may be regarded as a ground of the measurement unit.

The arrangement further comprises a galvanic connection, e.g. a cable, wherein the galvanic connection is adapted to interconnect the measurement units. The cable may be used as a common ground of the measurement units facilitating measurement of a resistance and/or impedance between the first contact and the second contact. The galvanic connection may be arranged within a hearing device, e.g. for the case that both contacts are arranged on a surface of a shell of the hearing device. Likewise, the galvanic connection may be a cable adapted to connect two hearing devices.

The contact resistance of the first electrode may be indicative of a resistance between the body and the first electrode. The contact resistance of the second electrode may be indicative of a resistance between the body and the second electrode.

The contact resistance may be a transition resistance which leads to a voltage drop, when a current passes from the electrode to a tissue of the body at the position where the electrode touches the body, in particular to a skin of the body. It may depend on multiple factors, for example the area of the electrodes and/or the force which is applied to press the contact against the skin. The contact resistance may also depend on the presence of sweat, cerumen or any other substance at the surface of the skin. The contact resistance may further depend on the material of the electrodes, on dirt, corrosion or an oxide formed at the surface of the electrode and on the roughness of the surface of the electrodes. The contact resistance needs to be taken into account in order to obtain a precise measurement of the body resistance between the two contacts. The contact resistance may depend also from the electrical configuration of the electrodes. For example both electrodes may be connected in parallel. **In** this case, the contact resistance may be measured by measuring a resistance between the body and the first and second electrode. **In** another case, the current may be injected from the first electrode of the contact. **In** this case, the contact resistance may be measured by measuring a resistance between the body and the first electrode.

Measuring a resistance may comprise measuring an impedance. Generally, impedance is a complex valued generalization of the resistance.

**In** an exemplary embodiment, each contact is arranged on a respective shell of a hearing device, the shell configured to be placed within an ear canal of a user, wherein the electrodes are arranged on an outside of the shell so as to be in contact with ear canal tissue.

In an exemplary embodiment, the electrodes are respectively formed as an arc.

In an exemplary embodiment, the electrodes are respectively formed as a fork with two or more prongs, wherein the prongs of one electrode are intertwined with those of the other electrode.

In an exemplary embodiment, the arrangement may comprise two hearing devices configured to be placed within a left ear canal and a right ear canal of the user, wherein the cable is arranged to interconnect the hearing devices. This allows for measuring the head impedance or resistance. Hearing devices are suitable for doing this measurement as they are arranged in a suitable location within the ear, comprise a processor, are capable of performing electrical measurements, and comprise communication interfaces to submit measurement results, e.g. to a smart phone. As a hearing device also comprises a speaker or receiver, it may easily be configured to acoustically inform the person wearing the hearing devices about the measurement or output a warning. The cable may be releasably connectable or connected to the hearing devices, e.g. by sockets and plugs.

In an exemplary embodiment, the measurement unit is connected to one of the electrodes with a first terminal, wherein a first switch is arranged to selectively short the two electrodes to connect both electrodes to the first terminal of the measurement unit.

In an exemplary embodiment, a second switch s arranged to selectively connect the other one of the electrodes with a second terminal of the measurement unit.

In an exemplary embodiment, a third switch is arranged to selectively connect the second terminal of the measurement unit to the cable. This has the advantage that a measurement of a contact resistance of a contact or an electrode of a contact is not disturbed by an electrical connection to another part of the body.

In an exemplary embodiment, the contacts are placed on a respective printed circuit board. The printed circuit board may be a flexible printed-circuit board.

In an exemplary embodiment, the measurement unit for measuring a resistance or an impedance may comprise a current source and a voltmeter and/or a voltage source and an amperemeter. This way, one of voltage and current is known and the other can be determined in order to determine a resistance. The current source or voltage source may be coupled to a power source of the hearing device and/or be supplied with energy from the power source of the hearing device. This allows for doing without an additional current source or voltage source. The voltage of the voltage source should be less than 4 volts. This allows for using Lithium accumulators and prevents subjecting the body to excessively high voltages.

According to an aspect of the present invention, a method of determining an electrical property of a human or animal body between a first position and a second position of the body using an arrangement as described above is proposed, wherein the first contact is placed to contact the body at the first position and the second contact is placed to contact the body at the second position, wherein a measurement is performed by applying an electrical signal to the contacts and determining a voltage of the electrical signal and a current of the electrical signal flowing through the body between the contacts, wherein a contact resistance is determined for at least one of the electrodes of the first contact or a contact resistance is determined for at least one of the electrodes of the first contact and at least one of the electrodes of the second contact. The electrical signal may be a DC signal or an AC signal. Applying an electrical signal may comprise applying a voltage or injecting a current. In case a (known) voltage is applied, determining comprises measuring the current. If a (known) current is injected, determining comprises measuring the voltage.

In an exemplary embodiment, the measurement is repeated multiple times to monitor the electrical property of the body over time.

In an exemplary embodiment, the first switch is open and the second switch is closed to determine a serial total contact resistance between the two electrodes via the body tissue. The body tissue may be understood as the tissue touching the electrodes of a contact at the position of the contact. The body tissue may be a skin of the body.

In an exemplary embodiment, the first switches and the third switches are closed and the second switch associated with one of the contacts is closed while the second switch associated with the other one of the contacts is open, wherein an impedance and/or resistance between the two contacts is determined as an impedance and/or resistance measured by the measurement unit minus parallel total contact resistances of both contacts.

The switches may be semiconductor switches.

In an exemplary embodiment, the contacts are arranged on a respective shell of a hearing device, wherein the shell is placed within an ear canal of a user, wherein the body tissue is an ear canal tissue.

In an exemplary embodiment, the impedance between the two contacts is used to assess one or more of:

- - skin elasticity,

- water content of tissue,
- blood profusion/circulation/pressure,
- hydration,
- heart beat if the contacts are situated near enough to an artery,
- sweat,
- whether a hearing device is worn or not,
- proximity/contact, and
- stress/depression.

The geometry of an earpieces or shell for a hearing instrument is typically shaped to match an anatomy of an inner ear channel of a user. Those shells are constantly in contact with the skin when worn by the user. By installing sensors on the shell, valuable information may be retrieved from the skin property of the ear channel without the need of additional external devices. The present invention describes how the skin conductivity and head impedance can be measured and possible applications to apply this information.

As the present invention allows for determining the contact resistance of each contact, the measurement of the head impedance may be made independent of this contact resistance which may vary depending on whether sweat or cerumen is present.

Further scope of applicability of the present invention will become apparent from the detailed description given hereinafter. However, it should be understood that the detailed description and specific examples, while indicating preferred embodiments of the invention, are given by way of illustration only, since various changes and modifications within the scope of the invention will become apparent to those skilled in the art from this detailed description.

Brief Description of the Drawings

The present invention will become more fully understood from the detailed description given hereinbelow and the accompanying drawings which are given by way of illustration only, and thus, are not limitive of the present invention, and wherein:

Figure 1   is a schematic detail view of a body tissue of an ear canal and a shell of a hearing device,

Figure 2   is a schematic view of an exemplary embodiment of a pair of electrodes respectively formed as an arc,

Figure 3   is a schematic view of an exemplary embodiment of a pair of electrodes respectively formed as forks with intertwined prongs,

Figure 4   is a schematic view of a head with two contacts during determination of contact resistances,

Figure 5   is a schematic view of a head with two contacts during determination of a head impedance, and

Figure 6   is a schematic view of a further embodiment showing a head with two contacts during determination of a head impedance.

Corresponding parts are marked with the same reference symbols in all figures.

Detailed Description of Preferred Embodiments

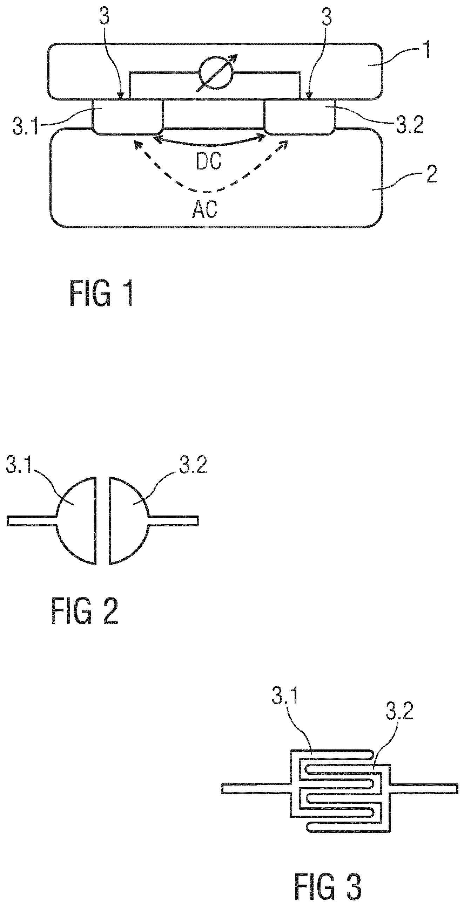

**Figure** 1 is a schematic detail view of a body tissue of an ear canal and a shell 1 of a hearing device 10. The shell 1 is placed into the ear canal and is thus located next to an ear canal tissue 2. A contact 3 is formed by two electrodes 3.1, 3.2 arranged on an outside of the shell 1 so as to be in contact with the ear canal tissue 2.

In order to measure the skin conductivity at a certain position of the ear canal for the monaural case, a direct voltage is applied between the electrodes 3.1, 3.2 and a resulting direct current DC between the electrodes 3.1, 3.2 can be measured. It is also possible to apply an alternating voltage between the electrodes 3.1, 3.2 and measure a resulting alternating current AC running through the ear canal tissue 2 between the electrodes 3.1, 3.2. A contact resistance can for example be determined from the DC-measurement. The AC-measurement may be used as an indicator for water content in the underlying tissue. Since water has a high relative dielectric constant the amount of water in a tissue has a strong impact on the AC-measurement. From the DC and AC measurements an impedance of the skin and the underlying tissue can be determined. The impedance is a complex value specifying the relation between the amplitudes of voltage and current of a sine wave signal with a frequency passing through an element.

The contact resistance depends on multiple factors which may vary over time: humidity, sweat, pressure, cerumen.

At a point in time t0 the conductivity of the skin at this precise position can thus be identified. By repeating the measurement, changes of the conductivity of the skin over a certain period T of time may be monitored.

In a binaural case, the said measurement can be performed in the left ear and in the right ear. Data obtained from both ears may be correlated, e.g. using a wireless connection, for example at 2.4 GHz, in order to provide a more accurate calculation. If, for example, the measurement of the contact resistance on the first ear differs by more than a predefined factor from the contact resistance of the contact of the second ear this may indicate, that the measurement conditions are not optimal for conducting a measurement of the head impedance. The predefined factor may be in the range of 1.1 to 5. In a preferred embodiment the predefined factor is in a range of 1.5 to 2. It should be pointed out that the contact resistance may be a real value specified in Ohm. It is also possible to determine a contact impedance, when an alternating current is applied to the contacts. In this case it is possible to specify the contact impedance as a complex value.

The knowledge of the contact resistance is necessary for precise (interaural) impedance measurements of the head or between any two positions at the body.

**Figure** 2 is a schematic view of an exemplary embodiment of a pair of electrodes 3.1, 3.2 respectively formed as an arc, also referred to as a segment of a circle.

**Figure** 3 is a schematic view of an exemplary embodiment of a pair of electrodes 3.1, 3.2 respectively formed as forks with two or more, e.g. three, prongs. The prongs of one electrode 3.1 are intertwined with those of the other electrode 3.2. This allows for averaging of local contact-resistance variations.

In order to measure a head impedance a cable 4 may be used to provide a galvanic interconnection of the contacts 3 formed by the electrodes 3.1, 3.2 for the left ear to those of the right ear.

The measurement may comprise determining the contact resistance between a respective pair of electrodes 3.1, 3.2 in both ears and determining the interaural impedance between the left ear and the right ear.

This measurement principle may also be applied to a measurement within an ear canal, e.g. two contacts 3 with respectively two electrodes 3.1, 3.2 are applied to different portions of the ear canal. The contact resistance for each pair of electrodes 3.1, 3.2 is determined and then the impedance between the two contacts 3 is determined.

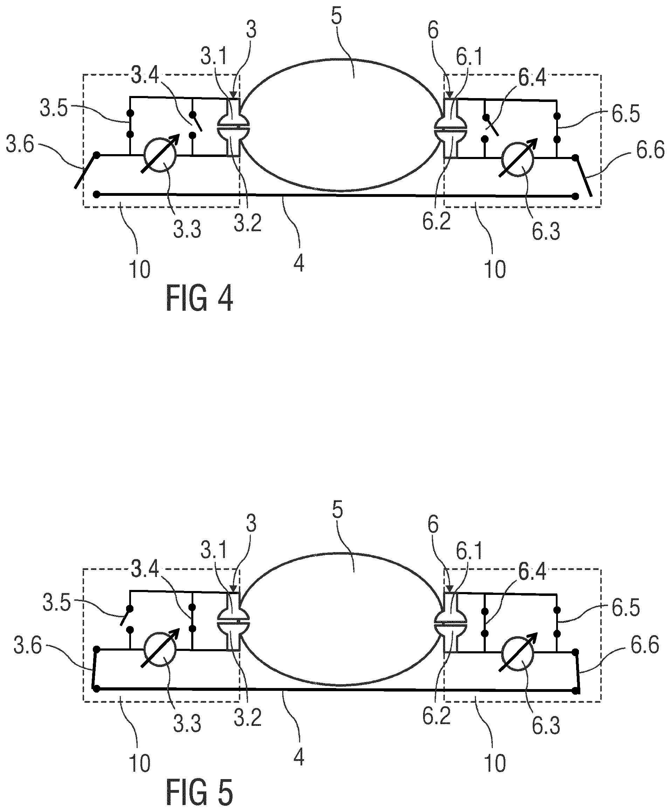

**Figure** 4 is a schematic view of a head 5 with two contacts 3, 6 during determination of contact resistances. The contacts 3, 6 may be applied within the left and right ear canal respectively. In figure 4 the contact resistance is determined between the two electrodes 3.1, 3.2 and 6.1, 6.2 of each contact 3 and 6 respectively. Each contact 3, 6 further comprises a measurement unit 3.3, 6.3 configured to measure an electrical resistance and/or impedance and three switches 3.4, 3.5, 3.6, 6.4, 6.5, 6.6. The measurement unit 3.3, 6.3 is connected to one of the electrodes 3.1, 6.1 with a first terminal. A first switch 3.4, 6.4 is configured to selectively short the two electrodes 3.1, 3.2, 6.1, 6.2 such that both electrodes 3.1, 3.2, 6.1, 6.2 are connected to the first terminal of the measurement unit 3.3, 6.3. A second switch 3.5, 6.5 is configured to selectively connect the other one of the electrodes 3.2, 6.2 with a second terminal of the measurement unit 3.3, 6.3. A third switch 3.6, 6.6 is configured to selectively connect the second terminal of the measurement unit 3.3, 6.3 to a cable 4 which may be used to connect the two contacts 3, 6. In figure 4 the third switches 3.6, 6.6 are open so that the contacts 3, 6 are not connected to each other. The skilled person readily understands that opening only one of the third switches 3.6, 6.6 in one of the contacts 3, 6 will suffice for this purpose. The first switch 3.4, 6.4 is open and the second switch 3.5, 6.5 is closed such that each terminal of the measurement unit 3.3, 6.3 is connected to one of the electrodes 3.1, 3.2, 6.1, 6.2 to determine a serial total contact resistance between the two electrodes 3.1, 3.2 or 6.1, 6.2 via the ear canal tissue 2 which is equal to the sum of the individual contact resistance of one of the electrodes 3.1, 6.1 to the ear canal tissue 2 and the individual contact resistance of the other one of the electrodes 3.2, 6.2 to the ear canal tissue 2 as these individual contact resistances are in series.

**Figure** 5 is a schematic view of a head 5 with two contacts 3, 6 during determination of the head impedance. In figure 5 both electrodes 3.1, 3.2 and 6.1, 6.2 of each contact 3, 6 are used in parallel. For this purpose the first switch 3.4, 6.4 is closed connecting both electrodes 3.1, 3.2, 6.1, 6.2 of each contact 3, 6. The third switches 3.6, 6.6 are closed interconnecting both contacts 3, 6. The skilled person readily understands that this may be achieved by only one third switch 3.6, 6.6 in one of the contacts 3, 6 while the other contact 3, 6 could be connected to the cable 4 without a third switch 3.6, 6.6. In one contact 6 the second switch 6.5 is closed thus shorting the measurement unit 6.3 and connecting the electrodes 6.1, 6.2 to the cable 4. In the other contact 3 the second switch 3.5 is open connecting the electrodes 3.1, 3.2 to the cable 4 through the measurement unit 3.3.

Each contact 3, 6 now contacts the ear canal tissue 2 with the two individual contact resistances in parallel. Assuming for the sake of simplicity that both individual contact resistances as discussed above with regard to figure 4 are equal within each contact 3, 6, i.e. half the serial total contact resistance, the parallel total contact resistance is half the individual contact resistance or a quarter of the serial total contact resistance.

The head impedance is thus equal to the impedance measured by the measurement unit 3.3 in the setup of figure 5 minus the parallel total contact resistances of both contacts 3, 6.

In particular with the fork-shaped electrodes 3.1, 3.2 as shown in figure 3 being used for both contacts 3, 6, equal individual contact resistances may be a save assumption. If there is any doubt that the individual contact resistances within one contact 3, 6 are equal, the individual contact resistances may be determined as follows:

Starting from the setting of figure 5 the first switch 3.4, 6.4 of one contact 3, 6 is opened and the measurement is repeated. As the electrodes 3.1, 3.2 or 6.1, 6.2 of that contact 3 or 6 are thus no longer switched in parallel but only one of them is part of the circuit the impedance measured will be increased with regard to the previous measurement by a difference accounting for the missing individual contact resistance. The knowledge of the serial total contact resistance as determined in the setup of figure 4 and the said difference allows for determining both individual contact resistances of the electrodes 3.1, 3.2, 6.1, 6.2 for the respective one of the contacts 3, 6 based on the relation that the reciprocal of the total resistance equals the sum of the reciprocals of the individual resistances in parallel circuits. Subsequently the first switch 3.4, 6.4 of said contact 3, 6 is closed again and the first switch 3.4, 6.4 of the other contact 3, 6 is opened to determine the individual contact resistances of the other contact 3, 6 in the same way.

The determination of the individual contact resistances may be renounced, if the measurement of the head impedance is high in comparison to the contact resistance. This may be the case for example if the head resistance is higher than any of the contact resistances by a factor of 10.

It should be pointed out, that a measurement unit for measuring a resistance or an impedance may comprise a current source and a voltmeter and/or a voltage source and an amperemeter. A resistance or the impedance can be calculated from the current and voltage values.

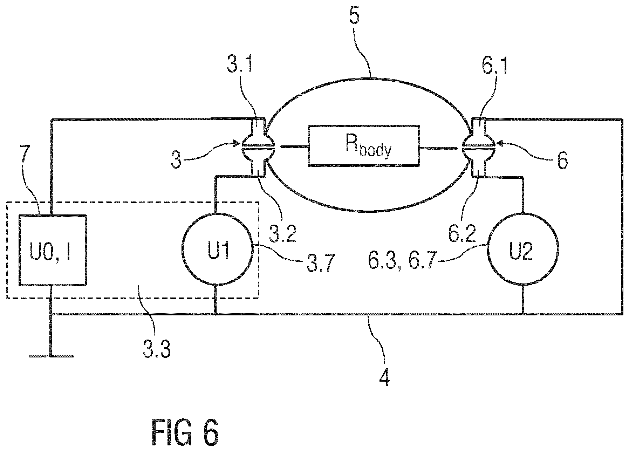

**Figure** 6 is a schematic view of a further embodiment. In this example an electrical source 7, which may be part of a measurement unit 3.3, 6.3, provides a voltage U0 and a current I. The current is injected into the first electrode 3.1 of the first contact 3. A first voltage U1 is measured at the second electrode 3.2 of the first contact 3 by the measurement unit 3.3, e.g. by a voltmeter 3.7 comprised in the measurement unit 3.3. The first electrode 6.1 of the second contact 6 is connected to the cable 4 which provides ground. A second voltage U2 is measured at the second electrode 6.2 of the second contact 6 by the measurement unit 6.3, e.g. by a voltmeter 6.7 comprised in the measurement unit 6.3. The measuring current of the measurement units 3.3, 6.3 is assumed to be neglectable. It may also be assumed, that the resistance between the first electrode 3.1 and the second electrode 3.2 of the first contact 3 is small in comparison to the resistance of the body between the first contact 3 and the second contact 6. It may also be assumed, that the resistance between the first and the second electrodes 6.1, 6.2 of the second contact 6 is small in comparison to the resistance of the body between the first contact 3 and the second contact 6.

The contact resistance Rc1 for the first contact 3 is then

$$Rc1 = (U0\text{-}U1) / I.$$

The contact resistance Rc2 for the second contact 6 is

$$Rc2 = U2 / I.$$

The resistance $R_{body}$ of the body or head 5 between the contacts 3, 6 is $R_{body} = (U1\text{-}U2) / I$.

From a mechanical point of view, both contacts 3, 6 can be placed on a printed circuit board (PCB). The PCB may be a flexible PCB. The contacts can be arranged also on the outer surface of an earpiece, in particular on the outer surface of a hearing device 10. Using a PCB may reduce the cost of production of the arrangement. A flexible PCB may add flexibility in the mechanical arrangement of the contacts.

The skin conductivity, the impedance between contacts in an ear canal, and the head or body impedance measurement may be used to assess:

- skin elasticity
- water content of tissue
- blood profusion/circulation/pressure
- hydration
- heart beat if the contacts 3 are situated near enough to an artery
- sweat
- whether a hearing device 10 is worn or not

- proximity/contact
- stress/depression

Skin elasticity, blood profusion and/or skin conductivity may be taken into account when modelling shells for hearing devices to improve the comfort of the shell in the ear.

List of References

| 1 | shell |
|---|---|
| 2 | ear canal tissue |
| 3 | first contact |
| 3.1 | electrode, first electrode |
| 3.2 | electrode, second electrode |
| 3.3 | measurement unit |
| 3.4 | first switch |
| 3.5 | second switch |
| 3.6 | third switch |
| 3.7 | voltmeter |
| 4 | cable |
| 5 | head |
| 6 | second contact |
| 6.1 | electrode, first electrode |
| 6.2 | electrode, second electrode |
| 6.3 | measurement unit |
| 6.4 | first switch |
| 6.5 | second switch |
| 6.6 | third switch |
| 6.7 | voltmeter |
| 7 | electrical source |
| 10 | hearing device |
| AC | alternating current |
| DC | direct current |
| U0, U1, U2 | voltage |
| I | current |
| $R_{body}$ | resistance |

**Claims**

1. An arrangement for measuring an electrical property of a human or animal body between a first position and a second position of the body, the arrangement comprising a first contact (3) configured to contact the body at the first position and a second contact (6) configured to contact the body at the second position, each contact (3, 6) comprising a first and a second electrode (3.1, 3.2, 6.1, 6.2) for contacting the body, the arrangement further comprising two measurement units (3.3, 6.6) configured to measure a resistance and/or impedance between the contacts (3, 6), wherein each one of the two measurements units is configured to measure a contact resistance between the two electrodes (3.1, 3.2, 6.1, 6.2) of a respective one of the two contacts, and a galvanic connection adapted to interconnect the measurement units (3.3, 6.3).

2. The arrangement of claim 1, wherein each contact (3, 6) is arranged on a respective shell (1) of at least one hearing device (10), the shell (1) configured to be placed within an ear canal of a user, wherein the electrodes (3.1, 3.2, 6.1, 6.2) are arranged on an outside of the shell (1) so as to be in contact with ear canal tissue (2).

3. The arrangement of claim 1 or 2, wherein the electrodes (3.1, 3.2, 6.1, 6.2) are respectively formed as an arc.

4. The arrangement of claim 1 or 2, wherein the electrodes (3.1, 3.2, 6.1, 6.2) are respectively formed as a fork with two or more prongs, wherein the prongs of one electrode (3.1, 6.1) are intertwined with those of the other electrode (3.2, 6.2).

5. The arrangement according to any one of claims 2 to 4, comprising two hearing devices (10) configured to be placed within a left ear canal and a right ear canal of the user, wherein the galvanic connection is configured as a cable (4) arranged to interconnect the hearing devices (10).

6. The arrangement according to any one of the preceding claims, wherein the measurement unit (3.3, 6.3) is connected to one of the electrodes (3.1, 6.1) with a first terminal, wherein a first switch (3.4, 6.4) is arranged to selectively short the two electrodes (3.1, 3.2, 6.1, 6.2) to connect both electrodes (3.1, 3.2, 6.1, 6.2) to the first terminal of the measurement unit (3.3, 6.3).

7. The arrangement according to claim 6, wherein a second switch (3.5, 6.5) is arranged to selectively connect the other one of the electrodes (3.2, 6.2) with a second terminal of the measurement unit (3.3, 6.3).

8. The arrangement according to claim 7, wherein a third switch (3.6, 6.6) is arranged to connect or disconnect the second terminal of the measurement unit (3.3, 6.3) to the cable (4).

9. The arrangement according to any one of claims 1 to 8, wherein the first measurement unit (3.3) comprises an electrical source (7) and a voltmeter (3.7), and the second measurement unit (6.3) comprises a voltmeter (6.7), wherein the electrical source (7) is connected to the cable (4) and to the first electrode (3.1) of the first contact (3), and the second electrode (3.2) of the first contact (3) is connected to the voltmeter (3.7) of the first measurement unit (3.3), and the cable (4) is connected to the first electrode (6.1) of the second contact (6), and the voltmeter (6.7) of the second measurement (6.3) unit is connected to the second electrode (6.3) of the second contact (6) and to the cable (4).

10. A method of determining an electrical property of a human or animal body between a first position and a second position of the body using an arrangement according to any one of the preceding claims, wherein the first contact (3) is placed to contact the body at the first position and the second contact (6) is placed to contact the body at the second position, wherein a measurement is performed by applying an electrical signal to the contacts and determining a voltage of the electrical signal and a current of the electrical signal flowing through the body between the contacts (3, 6), wherein a contact resistance is determined for at least one of the electrodes (3.1, 3.2) of the first contact (3, 6) or a contact resistance is determined for at least one of the electrodes (3.1, 3.2) of the first contact (3) and at least one of the electrodes (6.1, 6.2) of the second contact (6).

11. The method of claim 10, wherein the measurement is repeated multiple times to monitor the electrical property of the body over time.

12. The method according to claim 10 or 11, wherein the first switch (3.4, 6.4) is open and the second switch (3.5, 6.5) is closed to determine a serial total contact resistance between the two electrodes (3.1, 3.2, 6.1, 6.2) via the body tissue.

13. The method according to any one of claims 10 to 12, wherein the first switches (3.4, 6.4) and the third switches (3.6, 6.6) are closed and the second switch (3.5) associated with one of the contacts (3) is closed while the second switch (6.5) associated with the other one of the contacts (6) is open, wherein an impedance and/or a resistance between the two contacts (3, 6) is determined as an impedance and/or a resistance measured by the measurement unit (3.3, 6.3) minus parallel total contact resistances of both contacts (3, 6).

14. The method according to claim 13, wherein the contacts (3, 6) are arranged on a respective shell (1) of a hearing device (10), wherein the shell (1) is placed within an ear canal of a user, wherein the body tissue is an ear canal tissue (2).

15. The method according to claim 14, wherein the impedance between the two contacts (3, 6) is used to assess one or more of:

   - - skin elasticity,

      - water content of tissue,
      - blood profusion/circulation/pressure,
      - hydration,

- heart beat if the contacts (3, 6) are situated near enough to an artery,
- sweat,
- whether a hearing device (10) is worn or not,
- proximity/contact, and
- stress/depression.

**Patentansprüche**

1. Anordnung zum Messen einer elektrischen Eigenschaft eines menschlichen oder tierischen Körpers zwischen einer ersten Position und einer zweiten Position des Körpers, wobei die Anordnung einen ersten Kontakt (3), der dazu ausgelegt ist, den Körper an der ersten Position zu kontaktieren, und einen zweiten Kontakt (6), der dazu ausgelegt ist, den Körper an der zweiten Position zu kontaktieren, umfasst, wobei jeder Kontakt (3, 6) eine erste und eine zweite Elektrode (3.1, 3.2, 6.1, 6.2) zum Kontaktieren des Körpers umfasst, wobei die Anordnung ferner zwei Messeinheiten umfasst (3.3, 6.6), die dazu ausgelegt ist, einen Widerstand und/oder eine Impedanz zwischen den Kontakten (3, 6) zu messen, wobei jede der zwei Messeinheiten dazu ausgelegt ist, einen Kontaktwiderstand zwischen den zwei Elektroden (3.1, 3.2, 6.1, 6.2) eines jeweiligen der zwei Kontakte zu messen, und eine galvanische Verbindung, die dazu eingerichtet ist, die Messeinheiten (3.3, 6.3) zu verschalten.

2. Anordnung nach Anspruch 1, wobei jeder Kontakt (3, 6) auf einer jeweiligen Schale (1) mindestens einer Hörvorrichtung (10) angeordnet ist, wobei die Schale (1) dazu ausgelegt ist, innerhalb eines Gehörgangs eines Benutzers platziert zu werden, wobei die Elektroden (3.1, 3.2, 6.1, 6.2) auf einer Außenseite der Schale (1) angeordnet sind, um in Kontakt mit Gehörgangsgewebe (2) zu sein.

3. Anordnung nach Anspruch 1 oder 2, wobei die Elektroden (3.1, 3.2, 6.1, 6.2) jeweils als ein Bogen ausgebildet sind.

4. Anordnung nach Anspruch 1 oder 2, wobei die Elektroden (3.1, 3.2, 6.1, 6.2) jeweils als eine Gabel mit zwei oder mehr Zinken ausgebildet sind, wobei die Zinken einer Elektrode (3.1, 6.1) mit denen der anderen Elektrode (3.2, 6.2) verschlungen sind.

5. Anordnung nach einem der Ansprüche 2 bis 4, umfassend zwei Hörvorrichtungen (10), die dazu ausgelegt sind, innerhalb eines linken Gehörgangs und eines rechten Gehörgangs des Benutzers platziert zu werden, wobei die galvanische Verbindung als ein Kabel (4) ausgelegt ist, das dazu angeordnet ist, die Hörvorrichtungen (10) miteinander zu verschalten.

6. Anordnung nach einem der vorhergehenden Ansprüche, wobei die Messeinheit (3.3, 6.3) mit einem ersten Anschluss mit einer der Elektroden (3.1, 6.1) verbunden ist, wobei ein erster Schalter (3.4, 6.4) angeordnet ist, um die zwei Elektroden (3.1, 3.2, 6.1, 6.2) selektiv kurzzuschließen, um beide Elektroden (3.1, 3.2, 6.1, 6.2) mit dem ersten Anschluss der Messeinheit (3.3, 6.3) zu verbinden.

7. Anordnung nach Anspruch 6, wobei ein zweiter Schalter (3.5, 6.5) angeordnet ist, um selektiv die andere der Elektroden (3.2, 6.2) mit einem zweiten Anschluss der Messeinheit (3.3, 6.3) zu verbinden.

8. Anordnung nach Anspruch 7, wobei ein dritter Schalter (3.6, 6.6) angeordnet ist, um den zweiten Anschluss der Messeinheit (3.3, 6.3) mit dem Kabel (4) zu verbinden oder davon zu trennen.

9. Anordnung nach einem der Ansprüche 1 bis 8, wobei die erste Messeinheit (3.3) eine Stromquelle (7) und ein Voltmeter (3.7) umfasst, und die zweite Messeinheit (6.3) ein Voltmeter (6.7) umfasst, wobei die Stromquelle (7) mit dem Kabel (4) und mit der ersten Elektrode (3.1) des ersten Kontakts (3) verbunden ist, und die zweite Elektrode (3.2) des ersten Kontakts (3) mit dem Voltmeter (3.7) der ersten Messeinheit (3.3) verbunden ist, und das Kabel (4) mit der ersten Elektrode (6.1) des zweiten Kontakts (6) verbunden ist, und das Voltmeter (6.7) der zweiten Messeinheit (6.3) mit der zweiten Elektrode (6.3) des zweiten Kontakts (6) und mit dem Kabel (4) verbunden ist.

10. Verfahren zum Bestimmen einer elektrischen Eigenschaft eines menschlichen oder tierischen Körpers zwischen einer ersten Position und einer zweiten Position des Körpers unter Verwendung einer Anordnung nach einem der vorhergehenden Ansprüche, wobei der erste Kontakt (3) platziert wird, um den Körper an der ersten Position zu kontaktieren, und der zweite Kontakt (6) platziert wird, um den Körper an der zweiten Position zu kontaktieren, wobei eine Messung durch Anlegen eines elektrischen Signals an die Kontakte und Bestimmen einer Spannung des elektrischen Signals und eines Stroms des elektrischen Signals, das durch den Körper zwischen den Kontakten (3, 6) fließt, durchgeführt wird, wobei ein Kontaktwiderstand für mindestens eine der Elektroden (3.1, 3.2) des ersten Kontakts (3, 6) bestimmt wird oder ein Kontaktwiderstand für mindestens eine der Elektroden (3.1, 3.2) des ersten Kontakts (3) und mindestens einer der Elektroden (6.1, 6.2) des zweiten Kontakts (6) bestimmt wird.

11. Verfahren nach Anspruch 10, wobei die Messung mehrfach wiederholt wird, um die elektrische Eigenschaft des Körpers im Zeitverlauf zu überwachen.

**12.** Verfahren nach Anspruch 10 oder 11, wobei der erste Schalter (3.4, 6.4) offen ist, und der zweite Schalter (3.5, 6.5) geschlossen ist, um einen seriellen Gesamtkontaktwiderstand zwischen den zwei Elektroden (3.1, 3.2, 6.1, 6.2) über das Körpergewebe zu bestimmen.

**13.** Verfahren nach einem der Ansprüche 10 bis 12, wobei die ersten Schalter (3.4, 6.4) und die dritten Schalter (3.6, 6.6) geschlossen sind, und der zweite Schalter (3.5), der mit einem der Kontakte (3) assoziiert ist, geschlossen ist, während der zweite Schalter (6.5), der mit dem anderen der Kontakte (6) assoziiert ist, offen ist, wobei eine Impedanz und/oder ein Widerstand zwischen den zwei Kontakten (3, 6) als eine Impedanz und/oder ein Widerstand bestimmt wird/werden, die/der durch die Messeinheit (3.3, 6.3) gemessen wird, abzüglich paralleler Gesamtkontaktwiderstände beider Kontakte (3, 6).

**14.** Verfahren nach Anspruch 13, wobei die Kontakte (3, 6) auf einer jeweiligen Schale (1) einer Hörvorrichtung (10) angeordnet sind, wobei die Schale (1) in einem Gehörgang eines Benutzers platziert ist, wobei das Körpergewebe ein Gehörgangsgewebe (2) ist.

**15.** Verfahren nach Anspruch 14, wobei die Impedanz zwischen den zwei Kontakten (3, 6) verwendet wird, um eines oder mehrere von Folgendem zu bewerten:

- Hautelastizität,
- Wassergehalt des Gewebes,
- Blutfülle/Durchblutung/Blutdruck,
- Hydratation,
- Herzschlag, wenn die Kontakte (3, 6) nahe genug an einer Arterie liegen,
- Schweiß,
- ob ein Hörgerät (10) getragen wird oder nicht,
- Nähe/Kontakt, und
- Stress/Depression.

## Revendications

**1.** Agencement permettant de mesurer une propriété électrique d'un corps humain ou animal entre une première position et une seconde position du corps, l'agencement comprenant un premier contact (3) configuré pour entrer en contact avec le corps au niveau de la première position et un second contact (6) configuré pour entrer en contact avec le corps au niveau de la seconde position, chaque contact (3, 6) comprenant une première et une seconde électrode (3.1, 3.2, 6.1, 6.2) pour entrer en contact avec le corps, l'agencement comprenant en outre deux unités de mesure (3.3, 6.6) configurées pour mesurer une résistance et/ou une impédance entre les contacts (3, 6), chacune des deux unités de mesure étant configurée pour mesurer une résistance de contact entre les deux électrodes (3.1, 3.2, 6.1, 6.2) d'un contact respectif des deux contacts, et une connexion galvanique adaptée pour interconnecter les unités de mesure (3.3, 6.3).

**2.** Agencement selon la revendication 1, chaque contact (3, 6) étant agencé sur une coque respective (1) d'au moins un dispositif auditif (10), la coque (1) étant configurée pour être placée à l'intérieur d'un conduit auditif d'un utilisateur, les électrodes (3.1, 3.2, 6.1, 6.2) étant agencées sur une face extérieure de la coque (1) de manière à entrer en contact avec le tissu de conduit auditif (2).

**3.** Agencement selon la revendication 1 ou 2, les électrodes (3.1, 3.2, 6.1, 6.2) étant respectivement formées sous la forme d'un arc.

**4.** Agencement selon la revendication 1 ou 2, les électrodes (3.1, 3.2, 6.1, 6.2) étant respectivement formées sous la forme d'une fourche avec deux ou plus de deux dents, les dents d'une électrode (3.1, 6.1) étant entrelacées avec celles de l'autre électrode (3.2, 6.2).

**5.** Agencement selon l'une quelconque des revendications 2 à 4, comprenant deux dispositifs auditifs (10) configurés pour être placés à l'intérieur d'un conduit auditif gauche et d'un conduit auditif droit de l'utilisateur, la connexion galvanique étant configurée comme un câble (4) agencé pour interconnecter les appareils auditifs (10).

**6.** Agencement selon l'une quelconque des revendications précédentes, l'unité de mesure (3.3, 6.3) étant connectée à l'une des électrodes (3.1, 6.1) avec une première borne, un premier commutateur (3.4, 6.4) étant agencé pour court-circuiter sélectivement les deux électrodes (3.1, 3.2, 6.1, 6.2) afin de connecter les deux électrodes (3.1, 3.2, 6.1, 6.2) à la première borne de l'unité de mesure (3.3, 6.3).

**7.** Agencement selon la revendication 6, un deuxième commutateur (3.5, 6.5) étant agencé pour connecter sélectivement l'autre des électrodes (3.2, 6.2) à une seconde borne de l'unité de mesure (3.3, 6.3).

**8.** Agencement selon la revendication 7, un troisième commutateur (3.6, 6.6) étant agencé pour connecter ou déconnecter la seconde borne de l'unité de mesure (3.3, 6.3) au câble (4).

**9.** Agencement selon l'une quelconque des revendications 1 à 8, la première unité de mesure (3.3) comprenant une source électrique (7) et un voltmètre (3.7) et la seconde unité de mesure (6.3) comprenant un voltmètre (6.7), la source électrique (7) étant connectée au câble (4) et à la première électrode (3.1) du premier contact (3), la seconde électrode (3.2) du premier contact (3) étant connectée au voltmètre (3.7) de la première unité de mesure (3.3), et le câble (4) étant connecté à la première électrode (6.1) du second contact (6), et le voltmètre (6.7) de la seconde unité de mesure (6.3) étant connecté à la seconde électrode (6.3) du second contact (6) et au câble (4).

10. Procédé de détermination d'une propriété électrique d'un corps humain ou animal entre une première position et une seconde position du corps en utilisant un agencement selon l'une quelconque des revendications précédentes, le premier contact (3) étant placé pour entrer en contact avec le corps au niveau de la première position et le second contact (6) étant placé pour entrer en contact avec le corps au niveau de la seconde position, une mesure étant réalisée par application d'un signal électrique aux contacts et par détermination d'une tension du signal électrique et d'un courant du signal électrique circulant à travers le corps entre les contacts (3, 6), une résistance de contact étant déterminée pour au moins l'une des électrodes (3.1, 3.2) du premier contact (3, 6) ou une résistance de contact étant déterminée pour au moins l'une des électrodes (3.1, 3.2) du premier contact (3) et pour au moins l'une des électrodes (6.1, 6.2) du second contact (6).

11. Procédé selon la revendication 10, la mesure étant répétée plusieurs fois pour surveiller la propriété électrique du corps au fil du temps.

12. Procédé selon la revendication 10 ou 11, le premier commutateur (3.4, 6.4) étant ouvert et le deuxième commutateur (3.5, 6.5) étant fermé pour déterminer une résistance de contact totale en série entre les deux électrodes (3.1, 3.2, 6.1, 6.2) via le tissu corporel.

13. Procédé selon l'une quelconque des revendications 10 à 12, les premiers commutateurs (3.4, 6.4) et les troisièmes commutateurs (3.6, 6.6) étant fermés et le deuxième commutateur (3.5) associé à l'un des contacts (3) étant fermé tandis que le deuxième commutateur (6.5) associé à l'autre des contacts (6) est ouvert, une impédance et/ou une résistance entre les deux contacts (3, 6) étant déterminée comme l'impédance et/ou la résistance mesurée par l'unité de mesure (3.3, 6.3) moins les résistances de contact totales parallèles des deux contacts (3, 6).

14. Procédé selon la revendication 13, les contacts (3, 6) étant agencés sur une coque respective (1) d'un dispositif auditif (10), la coque (1) étant placée à l'intérieur d'un conduit auditif d'un utilisateur, le tissu corporel étant un tissu de conduit auditif (2).

15. Procédé selon la revendication 14, l'impédance entre les deux contacts (3, 6) étant utilisée pour évaluer le ou les des éléments suivants :

     - l'élasticité de la peau,
     - la teneur en eau des tissus,
     - la profusion/circulation/pression sanguine,
     - l'hydratation,
     - la fréquence cardiaque si les contacts (3, 6) sont situés suffisamment près d'une artère,
     - la transpiration,
     - le port ou non d'un dispositif auditif (10),
     - la proximité/le contact, et
     - le stress/la dépression.

FIG 1

FIG 2

FIG 3

FIG 4

FIG 5

FIG 6

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20170078785 A1

- EP 3154278 A1